# EUROPEAN PATENT APPLICATION

(11) **EP 4 696 335 A1**
(43) Date of publication of application: **18.02.2026**
(21) Application number: 25741644.6
(22) Date of filing: 17.01.2025
(51) Int. Cl.: A61L 15/36, D06M 16/00, A61F 13/15, A61F 13/84, A61L 15/24

(54) **PREPARATION METHOD FOR PROBIOTIC SANITARY PRODUCT**

(30) Priority: 19.01.2024 CN 202410083577
(71) Applicant: CHONGQING BAIYA SANITARY PRODUCTS CO., LTD., Chongqing 400000 (CN)
(72) Inventor: ZHANG, Xian, Chongqing 400000 (CN); MA, Chunyan, Chongqing 400000 (CN)
(74) Representative: Loo, Chi Ching
(86) International application number: PCT/CN2025/073122
(87) International publication number: WO 2025/153082

(57) **Abstract**

The present invention relates to the field of hygiene product production and discloses a method for preparing a probiotic hygiene product. The method includes the following steps: Step A, adding ultrapure water; Step B, adding an oil agent and performing stirring, including the following steps: B1, metering and adding the oil agent into the ultrapure water to form a mixed solution, where the oil agent is a hydrophilic oil agent; and B2, stirring the mixed solution; Step C, cooling the mixed solution; Step D, adding probiotic powder into the mixed solution to form a bacterial suspension, including the following steps: D1, metering and adding the probiotic powder into the mixed solution at one time to form a bacterial suspension, where the probiotic powder is probiotic bacterial powder (non-viable microbial type) containing an oil-soluble protective agent; and D2, stirring the bacterial suspension; Step E, coating fibers with the bacterial suspension; and Step F, producing a probiotic hygiene product using the fibers. This solution may solve the technical problem in the prior art that probiotics are unevenly distributed on the hygiene product when the hygiene product is prepared using live bacteria.

## Description

### TECHNICAL FIELD

The present invention relates to the field of hygiene product production, and in particular to, a method for preparing a probiotic hygiene product.

### BACKGROUND

Probiotics are active microorganisms by nature, and lactobacilli contained in the probiotics are the most abundant bacterial flora in a normal female vagina. In a healthy vaginal environment, the abundance of the lactobacilli may reach 97.56% to 99.99%, and the lactobacilli may generate substances such as lactic acid, hydrogen peroxide, and some enzymes, which are important guarantees for maintaining the stability of the vaginal microecological environment. Therefore, it is generally believed currently that adding probiotics is capable of achieving the purpose of enhancing the efficacy of sanitary napkins, maintaining the balance of vaginal floras, and keeping the female reproductive environment healthy. A probiotic sanitary napkin represents an upgrade to a fabric material of a conventional sanitary napkin. A top sheet containing probiotics in the sanitary napkin is a thermally-bonded non-woven fabric. The thermally-bonded non-woven fabric belongs to the category of thermally-bonded non-woven fabrics (including hot-rolled and thermally-bonded types). The thermally-bonded non-woven fabric is produced by first carding ordinary ES fibers into a web, then using hot air from drying equipment to penetrate the fiber web, and finally bonding the fibers through heat application.

A method for preparing a sanitary napkin containing vaginal probiotics (Publication No. CN106267319A) is disclosed in the prior art. The method includes two steps: preparing freeze-dried powder containing vaginal probiotics and uniformly distributing the freeze-dried powder in an absorption layer of the sanitary napkin. In the step of preparing the freeze-dried powder containing vaginal probiotics, materials are drawn within 3-7 days after the end of a menstrual period of a female using standardized gynecological examinations and vaginal secretion collection methods to prepare the freeze-dried powder containing vaginal probiotics, and then the freeze-dried powder is applied. The preparation process is tedious and is not applicable to an industrial production with a high requirement for production efficiency.

A female hygiene product containing probiotics and a preparation method therefor (Publication No. CN 102120045A) are further disclosed in the prior art. Water soluble probiotic freeze-dried powder is dissolved with sterile water to obtain a bacterial suspension with a total viable count of 1×109-1012 cfu/ml. Then the bacterial suspension is directly dispensed to a sanitary napkin, and the sanitary napkin is dried in a low-temperature condition (lower than 45°C) to obtain a finished product.

Technical problems in the prior art are as follows:
1. Although the prior art may be applied to an industrial production, strict regulatory indicators are currently imposed for the antibacterial rate of sanitary napkin products. The inventors have performed tests by adopting this solution and found that the prepared sanitary napkins do not meet the requirements for the antibacterial rate of textiles specified in the current standard GB/T 20944.3-2008; and the root cause lies in that during a reproduction and metabolism process, live bacteria tend to accumulate due to activity thereof, which leads to uneven distribution of probiotics on the sanitary napkins, such that the antibacterial rate fails to meet the standard requirements.
2. The freeze-dried powder contains the live bacteria, and the live bacteria are directly dispensed to the sanitary napkins. To ensure the activity of the live bacteria, the production condition requirements of the sanitary napkins will be more stringent, which not only leads to high costs, but also may generate harmful substances, resulting in reduced safety in use.

### SUMMARY

The present invention is intended to provide a method for preparing a probiotic hygiene product to solve the technical problem in the prior art that probiotics are unevenly distributed on the hygiene product when the hygiene product is prepared using live bacteria.

To achieve the above objective, the present invention adopts the following technical solution:
A method for preparing a probiotic hygiene product includes the following preparation steps:
Step A, adding ultrapure water;
Step B, adding an oil agent and performing stirring, including the following steps:
   B1, metering and adding the oil agent into the ultrapure water to form a mixed solution, where the oil agent is a hydrophilic oil agent; and
   B2, stirring the mixed solution;
Step C, cooling the mixed solution;
Step D, adding probiotic powder into the mixed solution to form a bacterial suspension, including the following steps:
   D1, metering and adding the probiotic powder into the mixed solution at one time to form a bacterial suspension, where the probiotic powder is probiotic bacterial powder (non-viable microbial type) containing an oil-soluble protective agent; and
   D2, stirring the bacterial suspension;
Step E, coating fibers with the bacterial suspension; and
Step F, producing a probiotic hygiene product using the fibers.

The concept and principle of the present invention are as follows:
To solve the technical problem in the prior art that probiotics are not uniformly distributed when the sanitary napkins are prepared by using the live bacteria, the inventors have replaced the bacterial suspension with a bacterial suspension containing inactivated probiotics. The inactivated probiotics not only retain the original characteristics of the live bacteria, but also are free of the ability to continue growing and reproducing. Upon detection of the prepared hygiene products, the inventors have found that the enhancement on distribution uniformity of probiotics is limited. Moreover, the inventors have found that the solution of using water-soluble probiotic freeze-dried powder in the prior art tends to form defects on the surfaces of hygiene products. After several experiments, finally, the inventors creatively obtained the technical solution including the step of adding the probiotic bacterial powder containing the oil-soluble protective agent (non-viable microbial type) and the step of coating fibers with the bacterial suspension and producing a probiotic hygiene product using the fibers, which effectively solves the problem that non-uniform distribution of probiotics and defects.

The principle and advantages of this solution are as follows:
1. Compared with a solution in the prior art where the water-soluble probiotic freeze-dried powder is dissolved with sterile water, probiotic bacterial powder (non-viable microbial type) containing the oil-soluble protective agent is added into the mixed solution prepared from ultrapure water and the oil agent to form the bacterial suspension containing the inactivated probiotics. The inactivated probiotics, belonging to a kind of postbiotics, are more stable and safer compared with the live bacteria disclosed in the prior art. Diluted and dissolved with the mixed solution, the bacterial suspension exhibits an obvious antibacterial effect on Escherichia coli, Staphylococcus aureus, Candida albicans, and the like, which meets the requirements for the antibacterial properties of textiles specified in the standard GB/T 20944.3-2008.
2. The purpose of mixing the hydrophilic oil agent in the ultrapure water lies in that since the fibers have no hydrophilicity or are directly water-repellent and only have water-absorbing performance after the hydrophilic oil agent is added, the step of adding the hydrophilic oil agent is basically included in methods for preparing special products that need to have water-absorbing functions, such as sanitary napkins and tampons; the inventors have found that the oil agent itself is a substance with strong adhesiveness, which is beneficial to preventing the inactivated probiotics on the sanitary products from peeling off, and also has an impact on improving the antibacterial effect of the sanitary products. The probiotic bacterial powder containing the oil-soluble protective agent is oil-soluble bacterial powder, which may be better mixed with the oil agent. In comparison, the water-soluble probiotic freeze-dried powder used in the prior art is poorly miscible with the oil agent. When the fibers are coated with the bacterial suspension prepared from the water-soluble probiotic freeze-dried powder, the water-soluble probiotic freeze-dried powder and the oil agent on the surfaces of the fibers are prone to accumulating to form dot-like substances that adhere to the probiotic sanitary products. The dot-like substances are also known as surface defects commonly referred to in textile fabrics.
3. In the prior art, the bacterial suspension is directly applied or sprayed to the nonwoven fabrics used in the sanitary napkins. In this solution, the fibers are coated with the bacterial suspension, and then the probiotic hygiene products are produced by using the fibers. In this manner, the probiotics on the prepared sanitary napkins are distributed uniformly, and the probiotics on the hygiene products have strong adhesion and are not easy to lose, such that the quality of the formed hygiene products is ensured, and the antibacterial stability is better.
4. Experiments show that the probiotic hygiene products prepared by this preparation method not only have a significant effect on bacteriostasis, but also have a promoting effect on the growth of beneficial bacteria including *Lactobacillus gasseri. Lactobacillus gasseri* is capable of regulating the acid-base balance in the body, improving the healthy environment, and enhancing immunity, thereby effectively inhibiting the growth and reproduction of harmful bacteria and maintaining the vaginal environment, but *Lactobacillus gasseri* exhibits high susceptibility to diminution or complete elimination. By using the probiotic hygiene products prepared by this preparation method, the problem of recurrent gynecological inflammation in women can be fundamentally solved.
5. The steps included in this solution may be automatically operated through a machine. Used raw materials such as the oil agent and the probiotic powder are all industrial conventional materials. Compared with the preparation method including standardized gynecological examinations and vaginal secretion collection method steps in the prior art, the preparation process is not tedious and is more suitable for an industrial production with a high requirement for production efficiency.

Preferably, as an improvement, the step of adding ultrapure water includes:
A1, metering and adding the ultrapure water into a blending tank; and
A2, heating the blending tank to raise the temperature of the ultrapure water to 60-80°C, where in the heating process, the ultrapure water needs to be stirred all the time.

The beneficial effects are as follows: When mixed solutions are prepared using different types of oil agents, the required water temperatures are different; when the mixed solutions are mixed using the hydrophilic oil agent, the ultrapure water needs to be preheated to 60-80°C, and excessively high or low water temperatures will affect the emulsification process that occurs when the probiotic powder is added to the mixed solutions after the solutions are prepared. The purpose of continuous stirring during this heating period is to ensure the uniformity of water temperature throughout the blending tank.

Preferably, as an improvement, in the step of adding the oil agent and performing stirring in Step B, a mass of the oil agent in A1 is metered as 0.35-0.45% of a mass of the fibers to be coated, and in the step of adding the probiotic powder, a mass of the probiotic powder in A1 is metered as 0.5-2.5% of the mass of the fibers to be coated.

The beneficial effects are as follows: As the hydrophilic auxiliary agent, an excessively low or high content of the oil agent in fibers will cause adverse effects to the formed hygiene product. If the oil content of the fibers is too low, the antistatic property of the hygiene product will deteriorate, and the adhesion of the inactivated probiotics will be weakened, thereby affecting the antibacterial effect; if the oil content of the fibers is too high, the adhesiveness of the fibers will be increased, the frictional resistance will be increased, and the web-forming uniformity of the fibers will be affected. As a skin-friendly material, the hygiene product provides a poor experience to customers. Experiments show that when the mass of the oil agent is 0.35-0.45% of the mass of the fibers to be coated, and the mass of the probiotic powder is 0.5-2.5% of the mass of the fibers to be coated, the antibacterial effect and the experience of the formed sanitary product can be well balanced.

Preferably, as an improvement, in the step of cooling the mixed solution in Step C, the mixed solution is cooled to below 50°C.

The beneficial effects are as follows: When the mixed solution is not cooled, the function of the inactivated probiotics will be reduced. It is worth noting that the inactivated probiotics are not equivalent to dead bacteria. Dead bacteria have no efficacy, while the inactivated probiotics retain the original characteristics of the live bacteria but do not have the ability to grow and reproduce.

Preferably, as an improvement, the oil-soluble protective agent includes 10% of linolenic acid, 15% of trehalose, 5% of glycerol, 6% of lauryl alcohol, 2% of L-arginine, and 1% of conjugated linolenic acid based on mass fraction, and the balance of water.

The beneficial effects are as follows: In the oil-soluble protective agent containing the above components, unsaturated fatty acids and carbohydrates, as effective protective substances, form a flexible protective layer outside the cells and can be dissolved in oils from different sources at any addition ratio ranging from 0% to 20% (w/w). After dissolution, the probiotic powder shows no unstable states such as stratification, precipitation, or agglomeration, which effectively prevents the bacterial powder from forming defects on the hygiene product and has a positive impact on the surface quality and antibacterial rate of the formed hygiene product.

Preferably, as an improvement, in the step of stirring the bacterial suspension, a stirring speed is 6,000-12,000 rpm/min, and a stirring time is 60-70 min.

The beneficial effects are as follows: The purpose of stirring the bacterial suspension is to guarantee the emulsification stability of the bacterial suspension. When the stirring speed is too low, the bacterial suspension is not uniformly stirred. When the stirring speed is too high, bubbles are easily generated, such that the bacterial suspension is not stable enough, which further affects the antibacterial performance of the formed hygiene product.

Preferably, as an improvement, in the step of coating fibers with the bacterial suspension, the bacterial suspension is continuously stirred at a stirring speed of 40-50 rpm/min, and the bacterial suspension is subjected to insulation, with a temperature controlled at 40-45°C.

The beneficial effects are as follows: The purpose of continuously stirring the bacterial suspension in the step of coating the fibers with the bacterial suspension is to guarantee the uniformity of the inactivated probiotics in the bacterial suspension. The stirring speed is greatly decreased compared with that before coating. This is because the bacterial suspension is uniform enough before coating and the uniformity is maintained at a relatively low stirring speed. Setting the rotating speed to a high rotation speed would be still a waste of energy consumption.

Preferably, as an improvement, in the step of stirring the bacterial suspension, the stirring speed is 10,000 rpm/min, and the stirring time is 65 min.

The beneficial effects are as follows: When the stirring speed is 10,000 rpm/min and the stirring time is 65 min, the bacterial suspension can be fully emulsified, which maximizes the stability of the inactivated probiotics on the formed hygiene products and achieves the maximum increase in antibacterial rate.

Preferably, as an improvement, the probiotic hygiene product is a thermally-bonded non-woven fabric.

The beneficial effect is as follows: The production process for the thermally-bonded non-woven fabric is an advanced production process and has the advantages of high efficiency, high quality, and low cost.

Preferably, as an improvement, the top sheet, the acquisition layer, and a coating layer of the acquisition core are made of the thermally-bonded non-woven fabric.

The beneficial effects are as follows: The thermally-bonded non-woven fabric made by a hot-air penetration bonding process has characteristics such as bulkiness and softness, and also has high openness, air permeability, ductility, and strength. Moreover, compared with a common non-woven fabric, the thermally-bonded nonwoven fabric produced by the above preparation method has a higher antibacterial rate against bacterial and fungal strains such as Escherichia coli, Staphylococcus aureus, and Candida albicans. The top sheet, the acquisition layer, and the coating layer of the acquisition core of the female sanitary napkin that come into contact with the skin and are prone to blood staining exactly require these characteristics.

### BRIEF DESCRIPTION OF THE DRAWINGS

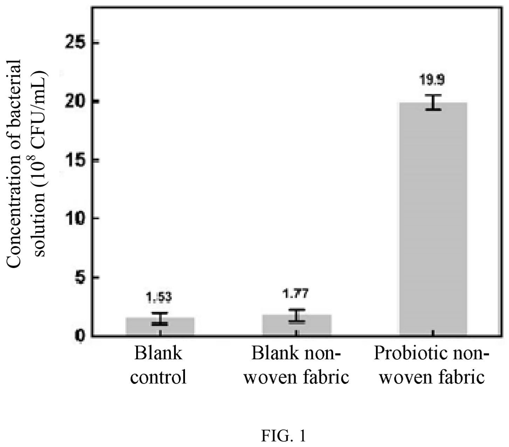

FIG. 1 is a graph showing test results of an efficacy in promoting growth of *Lactobacillus gasseri* for a probiotic non-woven fabric prepared in Example I and a blank non-woven fabric.

### DETAILED DESCRIPTION OF EMBODIMENTS

### Example I

A method for preparing a thermally-bonded non-woven fabric includes:
Step A, ultrapure water was added.
A1, the ultrapure water was metered and added into a blending tank; and
A2, the blending tank was heated to raise the temperature of the ultrapure water to 70°C, so as to guarantee mixing and emulsifying effects; and in the heating process, the ultrapure water was stirred all the time to ensure the water temperature in the whole blending tank uniform.

Step B, an oil agent was added and stirring was performed
B1, 4 kg of the oil agent was metered and added into the blending tank to form a mixed solution of the ultrapure water and the oil agent, where the specific oil agent used in this example was a hydrophilic oil agent (also known as a hydrophilic agent), which was an anionic surfactant containing phosphate, sulfonate, and succinate ester components, might be adsorbed on the surface of solid particles, improved the interaction force between particles, and had good hydrophilicity.
B2, the mixed solution of the ultrapure water and the oil agent was stirred, where a stirring speed was 100 rpm/min, and a stirring time was ≥30 min.

Step C, the mixed solution was cooled.

C1, the mixed solution was cooled to 50°C.

Step D, probiotic powder was added to form a bacterial suspension
D1, 12.5 kg of the probiotic powder was metered and added into the blending tank at one time to form a bacterial suspension; in this example, the probiotic powder was probiotic bacterial powder (non-viable microbial type) containing the oil-soluble protective agent, where the oil-soluble protective agent included 10% of linolenic acid, 15% of trehalose, 5% of glycerol, 6% of lauryl alcohol, 2% of L-arginine, 1% of conjugated linolenic acid, and the balance of water (based on mass fraction).
D2, the bacterial suspension was stirred, where a stirring speed was 10,000 rpm/min, and a stirring time was 65 min.

Step E, the fibers were coated with the bacterial suspension.
E1, the bacterial suspension in the blending tank was poured into a storage tank used for coating; and
E2, the fibers were coated, where in this example, the mass of the fibers to be coated was 1,000 kg; in the fiber coating process, the bacterial suspension was continuously stirred, a stirring speed was 45 rpm/min, the bacterial suspension was insulated, and a temperature was controlled at 43°C.

Step F, a thermally-bonded non-woven fabric was produced by using the fibers
F1, the fibers were fed into a hot-air oven for thermal bonding to form a thermally-bonded non-woven fabric (hereinafter collectively referred to as "a probiotic nonwoven fabric").

In examples except for this example, a female sanitary napkin is further disclosed, including a top sheet, an acquisition layer, and an acquisition core, where any one or more of the top sheet, the acquisition layer, and the acquisition core is made of the produced thermally-bonded non-woven fabric.

### Example II

The difference between this example and Example I lies in that in the step of adding the probiotic powder, the mass of the probiotic power in A1 is 0.5% of the mass of the fibers to be coated, i.e., 5 kg.

### Example III

The difference between this example and Example I lies in that in the step of adding the probiotic powder, the mass of the probiotic power in A1 is 2.5% of the mass of the fibers to be coated, i.e., 25 kg.

### Example IV

The difference between this example and Example I lies in that in the step of adding the probiotic powder, the mass of the probiotic power in A1 is 1% of the mass of the fibers to be coated, i.e., 10 kg.

### Example V

The difference between this example and Example I lies in that in the step of adding the probiotic powder, the mass of the probiotic power in A1 is 2% of the mass of the fibers to be coated, i.e., 20 kg.

### Comparative Example I

The difference between this comparative example and Example I lies in that in Step E, the bacterial suspension prepared in Step D is directly sprayed or applied to a product thermally-bonded non-woven fabric to form the probiotic non-woven fabric with the inactivated probiotics.

### Comparative Example II

The difference between this comparative example and Example I lies in that the probiotic powder added in Step IV is the water-soluble probiotic bacterial powder (non-viable microbial type) without the oil-soluble protective agent.

### Experimental Example

An antibacterial test was conducted on the probiotic non-woven fabric prepared in Example I, and the test method referred to GB/T 20944.3-2008 *Evaluation of Antibacterial Activity of Textiles - Part 3: Shake Flask Method.*

Test results are as follows:

**Table 1**

| Name of experimental strains (Number of strains) | *Escherichia coli* 8099 | *Staphylococcus aureus* ATCC 6538 | *Candida albicans* ATCC 10231 |
|---|---|---|---|
| Concentrations of inoculated bacterial solutions (CFU/ml) | 2.0*10⁴ | 2.1*10⁴ | 1.8*10⁴ |
| Growth value of control sample F (F=I_{g}Wₜ-I_{g}Wₚ) (Wₜ&W₀: CFU/ml) | 2.6 (Wₜ:7.5*10⁶ W₀:2.0*10⁴) | 2.4 (Wₜ:4.5*10⁶ W₀:2.1*10⁴) | 0.8 (W₁:1.2*10⁵ W₀:1.8*10⁴) |
| Live bacteria concentration in the flask after 18 h of oscillating contact with the antibacterial sample (Qₜ)(CFU/ml) | 2.1*10⁶ | 9.1*10⁵ | 4.5*10⁴ |
| Antibacterial rate (%) | 72 | 80 | 63 |
| Sterilization method | High-pressure steam sterilization at 121°C for 15 min | | |

According to the evaluation criteria of GB/T 20944.3-2008, the antibacterial rate of a sample against *Staphylococcus aureus* and *Escherichia coli* is ≥70%, and the sample is considered to have an antibacterial effect. The antibacterial rate of a sample against *Candida albicans* is ≥60%, and the sample is considered to have an antibacterial effect.

As shown in the results of Table 1, in terms of antibacterial activity, the antibacterial rate of the probiotic non-woven fabric in Example I against *Escherichia coli* (8099) reaches 72%, indicating an antibacterial effect; the antibacterial rate of Example I against *Staphylococcus aureus* (ATCC 6538) reaches 80%, indicating that the probiotic non-woven fabric in Example 1 has an antibacterial effect; the antibacterial rate of the probiotic non-woven fabric in Example I against *Candida albicans* (ATCC 10231) reaches 63%, indicating that the probiotic non-woven fabric in Example 1 has an antibacterial effect.

Antibacterial tests were conducted on the probiotic non-woven fabrics prepared in Example II, Example III, Example IV, Example V, Comparative Example I, and Comparative Example II, and the test method referred to GB/T 20944.3-2008 *Evaluation of Antibacterial Activity of Textiles - Part 3: Shake Flask Method.*

Test results on *Escherichia coil* 8099 are as follows:

**Table 2**

| | Example II | Example III | Example IV | Example V | Comparati ve Example I | Comparati ve Example II |
|---|---|---|---|---|---|---|
| Antibacteri al rate (%) | 70 | 71 | 73 | 71 | 52 | 62 |
| Evaluation | Antibacteri al effect | Antibacteri al effect | Antibacteri al effect | Antibacteri al effect | No antibacteri al effect | No antibacteri al effect |

Test results on *Staphylococcus aureus* ATCC6538 are as follows:

**Table 3**

| | Example II | Example III | Example IV | Example V | Comparati ve Example I | Comparati ve Example II |
|---|---|---|---|---|---|---|
| Antibacteri al rate (%) | 71 | 77 | 78 | 70 | 59 | 65 |
| Evaluation | Antibacteri al effect | Antibacteri al effect | Antibacteri al effect | Antibacteri al effect | No antibacteri al effect | No antibacteri al effect |

Test results on *Candida albicans* ATCC10231 are as follows:

**Table 4**

| | Example II | Example III | Example IV | Example V | Comparati ve Example I | Comparati ve Example II |
|---|---|---|---|---|---|---|
| Antibacteri al rate (%) | 62 | 60 | 61 | 60 | 43 | 54 |
| Evaluation | Antibacteri al effect | Antibacteri al effect | Antibacteri al effect | Antibacteri al effect | No antibacteri al effect | No antibacteri al effect |

It can be known from the test results of Table 2, Table 3, and Table 4 that the antibacterial rates of the probiotic non-woven fabric in Comparative Example I against *Escherichia coli, Staphylococcus aureus,* and *Candida albicans* are respectively 52%, 59%, and 43%, without an antibacterial effect. Through deep research, the root cause lies in that in Step E, the bacterial suspension prepared in Step D is directly sprayed or applied to the product thermally-bonded non-woven fabric. This step cannot guarantee the distribution uniformity of the probiotics on the nonwoven fabric, which causes adverse effects on the antibacterial rate of the prepared probiotic non-woven fabric.

The surfaces of the probiotic non-woven fabrics prepared in Example I, Example II, Example III, Example IV, Example V, and Comparative Example II are observed with naked eyes. The surfaces of the probiotic non-woven fabrics prepared in Examples I-V are smooth without obvious defects. The surface of the probiotic non-woven fabric prepared in Comparative Example II has obvious defects, which affect the antibacterial rate of the probiotic non-woven fabric. As shown in Tables 2-4, the antibacterial rates of the probiotic non-woven fabric in Comparative Example II against *Escherichia coli, Staphylococcus aureus,* and *Candida albicans* are respectively 62%, 65%, and 54%, without an antibacterial effect. Poor skinfriendliness during use is predictable and tends to result in an undesirable user experience.

A test on the efficacy of promoting the growth of *Lactobacillus gasseri* was conducted on the probiotic nonwoven fabric prepared in Example I and the blank nonwoven fabric (a common nonwoven fabric without probiotics added available on the market). The test method was a plate counting method, including the following operating steps:

### 1. Sample pre-treatment

A probiotic nonwoven fabric and a blank non-woven fabric were weighted, cut into 1 cm2 pieces, and placed in 100 mL of a liquid Modified Rogosa and Sharpe (MRA) medium for sterilization for later use.

### 2, Reagent preparation

The liquid MRA medium: 2% of glucose, 1% of lactose, 1% of peptone, 0.5% of yeast extract, 1% of beef extract powder, 0.2% of dipotassium hydrogen phosphate, 0.1% of Tween-80, 0.058% of magnesium sulfate, 0.019% of manganese sulfate, and 0.1% of L-cysteine;
A solid medium: 66.2 g of a product MRA medium was dissolved in 1,000 mL of pure water, and 0.1% of L-cysteine was added.

### 3. Strain activation

800 µL of *Lactobacillus gasseri* cells from the glycerol stock tube were transferred into a test tube containing 9 mL of the liquid MRA medium, and cultured at 37°C for 24 h. At an inoculum size of 2% (v/v), the above bacterial solution was taken and inoculated into the liquid MRA medium, cultured at 37°C for 16 h, and the bacterial suspension was retained for later use.

### 4. Strain co-cultivation

A *Lactobacillus gasseri* suspension was inoculated at an inoculum size of 2% (v/v) into the liquid MRA medium containing the probiotic nonwoven fabric, the liquid MRA medium containing blank nonwoven fabric, respectively. The blank control was the liquid MRA medium with 2% of the *Lactobacillus gasseri* suspension, and were all cultured at 37°C for 16 h.

### 5. Plate counting

A 10-fold serial dilution was performed, and 3 appropriate dilution levels were selected. 100 µL was taken from each of the co-cultures with three different dilution factors and evenly spread on a solid medium, followed by anaerobic incubation at 37°C for 48 h. After the cultivation, the growth of colonies on the 3 types of plates with different dilution factors was observed, and the cultured colonies were counted.

Test results are as follows:
As shown by the results in FIG. 1, the bacterial concentration in the *Lactobacillus gasseri* suspension is 1.53×10⁸ CFU/mL, the bacterial concentration in the Lactobacillus gasseri suspension containing the blank non-woven fabric reaches 1.77×108 CFU/mL, and the bacterial concentration in the *Lactobacillus gasseri* suspension containing the probiotic non-woven fabric reaches 1.90×10⁹ CFU/mL, indicating that the probiotic non-woven fabric has a certain effect of promoting growth of *Lactobacillus gasseri.*

The above descriptions are only the embodiments of the present invention, and common knowledge such as the known specific technical solutions and/or characteristics in the solutions are not described in detail herein. It should be noted that for those skilled in the art, several modifications and improvements may still be made without departing from the technical solution of the present invention, and these should also be deemed as the protection scope of the present invention, and none of these would affect the implementation effect of the present invention or the practicability of the patent. The scope of protection claimed in present application should be subject to the content of claims of the present application, and the records such as the specific implementation modes in the specification may be used to interpret the content of the claims.

## Claims

1. A method for preparing a probiotic hygiene product, **characterized by** comprising the following steps:
Step A, adding ultrapure water;
Step B, adding an oil agent and performing stirring, comprising the following steps:
B1, metering and adding the oil agent into the ultrapure water to form a mixed solution, wherein the oil agent is a hydrophilic oil agent; and
B2, stirring the mixed solution;
Step C, cooling the mixed solution;
Step D, adding probiotic powder into the mixed solution to form a bacterial suspension, comprising the following steps:
D1, metering and adding the probiotic powder into the mixed solution at one time to form a bacterial suspension, wherein the probiotic powder is probiotic bacterial powder (non-viable microbial type) containing an oil-soluble protective agent; and
D2, stirring the bacterial suspension;
Step E, coating fibers with the bacterial suspension; and
Step F, producing a probiotic hygiene product using the fibers.

2. The method for preparing a probiotic hygiene product according to claim 1, **characterized in that** the step of adding ultrapure water in Step A comprises:
A1, metering and adding the ultrapure water into a blending tank; and
A2, heating the blending tank to raise the temperature of the ultrapure water to 60-80°C, wherein in the heating process, the ultrapure water needs to be stirred all the time.

3. The method for preparing a probiotic hygiene product according to claim 2, **characterized in that** in the step of adding the oil agent and performing stirring in Step B, a mass of the oil agent in A1 is metered as 0.35-0.45% of a mass of the fibers to be coated, and in the step of adding the probiotic powder, a mass of the probiotic powder in A1 is metered as 0.5-2.5% of the mass of the fibers to be coated.

4. The method for preparing a probiotic hygiene product according to claim 3, **characterized in that** in the step of cooling the mixed solution in Step C, the mixed solution is cooled to below 50°C.

5. The method for preparing a probiotic hygiene product according to claim 4, **characterized in that** the oil-soluble protective agent used in Step D1 comprises 10% of linolenic acid, 15% of trehalose, 5% of glycerol, 6% of lauryl alcohol, 2% of L-arginine, and 1% of conjugated linolenic acid based on mass fraction, and the balance of water.

6. The method for preparing a probiotic hygiene product according to claim 5, **characterized in that** in the step of stirring the bacterial suspension in Step D2, a stirring speed is 6,000-12,000 rpm/min, and a stirring time is 60-70 min.

7. The method for preparing a probiotic hygiene product according to claim 6, **characterized in that** in the step of coating fibers with the bacterial suspension in step E, the bacterial suspension is continuously stirred at a stirring speed of 40-50 rpm/min, and the bacterial suspension is insulated, with a temperature controlled at 40-45°C.

8. The method for preparing a probiotic hygiene product according to claim 7, **characterized in that** in the step of stirring the bacterial suspension in Step D2, the stirring speed is 10,000 rpm/min, and the stirring time is 65 min.

9. The method for preparing a probiotic hygiene product according to claim 1, **characterized in that** in Step F, a thermally-bonded non-woven fabric is produced using the fibers, and then the thermally-bonded non-woven fabric is laminated and pressed into a sanitary napkin or diaper to form the probiotic hygiene product.

10. A female sanitary napkin, **characterized by** comprising a top sheet, an acquisition layer, and an acquisition core, wherein any one or more of the top sheet, an acquisition layer, and an acquisition core are made of the thermally-bonded nonwoven fabric according to claim 9.
